# FASCICULE DE BREVET EUROPEEN

(11) **EP 2 699 314 B1**
(45) Date de publication et mention de la délivrance du brevet: **06.01.2016**
(21) Numéro de dépôt: 12717269.0
(22) Date de dépôt: 20.04.2012
(51) Int. Cl.: A61N 5/10

(54) **DISPOSITIF MUNI D'UN JEU D'APPLICATEURS POUR LA RADIOTHÉRAPIE DE CONTACT ET SYSTÈME COMPRENANT LEDIT DISPOSITIF**
VORRICHTUNG MIT EINEM APPLIKATOR-SET FÜR KONTAKTSTRAHLENTHERAPIE UND SYSTEM MIT DIESER VORRICHTUNG
DEVICE PROVIDED WITH A SET OF APPLICATORS FOR CONTACT RADIOTHERAPY, AND SYSTEM COMPRISING SAID DEVICE

(30) Priorité: 22.04.2011 FR 1153529
(43) Date de publication de la demande: 26.02.2014
(73) Titulaire: Centre Antoine Lacassagne, 06189 Nice Cedex 2 (FR); Gerard, Jean-Pierre, 06000 Nice (FR)
(72) Inventeur: GERARD, Jean-Pierre, 06000 Nice (FR); MARCIE, Serge, 06700 Saint Laurent Du Var (FR); SPANSWICK, Keith Albert, Ravenshead, Nottinghamshire Ng15 9BE (GB); TRIMAUD, Richard, 06140 Vence (FR); FLIPO, Bernard, 06189 Nice Cedex 2 (FR); RAOUST, Inès, 06189 Nice Cedex 2 (FR)
(74) Mandataire: Brizio Delaporte, Allison
(86) Numéro de dépôt international: PCT/EP2012/057317
(87) Numéro de publication internationale: WO 2012/143530

(56) Documents cités:
- GB-A- 854 825
- US-A- 4 763 642
- US-A- 5 910 102
- US-A1- 2005 080 313
- US-A1- 2005 240 073
- Jack F. Fowler ET AL: "Biological effect of pulsed dose rate brachytherapy with stepping sources if short half-times of repair are present in tissues", International Journal of Radiation OncologyBiologyPhysics, vol. 37, no. 4, 1 March 1997 (1997-03-01), pages 877-883, XP055195220, ISSN: 0360-3016, DOI: 10.1016/S0360-3016(96)00565-2

## Description

La présente invention concerne un dispositif muni d'un jeu d'applicateurs pour la radiothérapie de contact et un système comprenant ledit dispositif.

La radiothérapie de contact est une technique d'irradiation en photons de faible tension. Elle est notamment destinée à l'irradiation de tumeurs cutanées, superficielles et endocavitaires.

La présente invention s'applique plus particulièrement à la radiothérapie per opératoire, c'est-à-dire que l'irradiation est réalisée au contact du lit d'exérèse de la tumeur notamment dans le sein au cours de l'opération. Pour cela, les dispositifs comprennent un applicateur qui est destiné à être au contact du tissu à traiter. Bien que la tumeur ait été retirée, des cellules cancéreuses peuvent être dispersées dans les proches tissus environnants. Une irradiation de ceux-ci permet de stériliser d'éventuelles cellules cancéreuses et de limiter les rechutes.

On connaît le document US- A1- 2005/0240073 qui décrit un dispositif pour la radiothérapie dont l'applicateur comprend un corps rigide ayant une tête, pouvant être en Téflon®, sensiblement sphérique et pleine. La tête comprend au moins un canal de faible section permettant le guidage lors de l'insertion de la source d'agent de traitement.

Dans la radiothérapie de contact, l'irradiation est de faible tension à action très superficielle. Cette irradiation a faible tension combinée à une tête pleine limite la dose produite par unité de temps, soit le débit de dose, et augmente d'autant les durées de traitement. De plus, l'irradiation est réalisée à proximité immédiate des tissus sains avec des doses très élevées. Il est donc particulièrement nécessaire de prévoir un dispositif capable de fournir une irradiation contrôlée et sécurisée sur une zone déterminée et ce quels que soient le patient et la zone à irradier de sorte à limiter les risques d'erreurs des opérateurs.

A cet effet, un aspect de l'invention concerne un dispositif muni d'une pluralité d'applicateurs de forme sensiblement sphérique et de diamètre différent de sorte à s'adapter à toutes les cavités à irradier. L'épaisseur de l'applicateur est une fonction du diamètre de l'applicateur telle qu'elle permet la délivrance d'une dose constante, homogène, de l'ordre de 18 à 22 Gray à la surface de l'applicateur et permet d'optimiser le rendement en profondeur.

De cette manière, quelle que soit la taille de la cavité à irradier, la tête de l'applicateur est au contact du tissu sain et une dose de rayons X connue et contrôlée est délivrée au tissu à irradier.

De manière très avantageuse, les applicateurs selon l'invention sont creux, ce qui améliore nettement le débit de dose à la surface de l'applicateur.

D'autres buts et avantages apparaîtront au cours de la description qui suit qui présente un mode de réalisation de l'invention illustratif mais non limitatif.

On rappelle tout d'abord que l'invention concerne un dispositif pour la radiothérapie de contact caractérisé par le fait qu'il comprend un jeu d'applicateurs dont chaque applicateur comprend une tête sphérique et creuse, de diamètre différent dont la surface extérieure est apte à être en contact avec une cavité d'un tissu vivant et dont la surface intérieure définit un volume intérieur apte à recevoir une source de rayons X, dans lequel au moins une zone de la tête est destinée à être traversée par des rayons X et l'épaisseur de ladite zone est une fonction du diamètre de la tête configurée de sorte que la dose de rayons X produite à la surface extérieure est comprise entre 18 et 22 Gray.

Suivant des variantes alternatives ou cumulatives préférées mais non limitatives, l'invention est telle que :
- l'épaisseur de ladite zone de la tête de chaque applicateur est comprise entre 1 et 3,5 millimètres préférentiellement entre 1 et 2,5 millimètres ;
- le diamètre de la tête de chaque applicateur est compris entre 2 et 7 centimètres, préférentiellement entre 2 et 6 centimètres ;
- le diamètre de la tête de chaque applicateur correspond à un secteur angulaire d'au moins 300° ;
- la tête de chaque applicateur comprend une matière plastique apte à être stérilisée;
- chaque applicateur comprend un rebord en partie supérieure de la tête d'application,
- chaque applicateur comprend un manche s'étendant depuis la tête d'application, ledit manche recevant le rebord configuré pour être mobile en translation le long du manche,
- chaque applicateur comprend des moyens de fixation de la tête dans la cavité d'un tissu vivant ;
- les moyens de fixation comprennent un rebord, muni de trous, formant une calotte sur la tête de l'applicateur ;
- la tête de chaque applicateur comprend une ouverture apte à permettre l'introduction de la source de rayons X dans le volume intérieur.

Suivant une autre possibilité, l'invention concerne également un système de radiothérapie de contact comprenant un dispositif tel que décrit ci-dessus et une source de rayons X émettant un faisceau isotrope.

Avantageusement, la source de rayons X est polychrome et émet une puissance de 30 à 80 Kv préférentiellement de 50 Kv à 70 Kv.

Avantageusement, la source de rayons X est disposée au centre de la tête de l'applicateur.

Avantageusement, le volume intérieur de la tête d'application est rempli de gaz, plus préférentiellement d'air.

Avantageusement, il comprend un filtre en aluminium agencé au niveau de la source de rayons X.

Avantageusement, le filtre a une épaisseur comprise entre 0,2 et 2,5 millimétres, préférentiellement de 0,3 millimètres.

Avantageusement, le débit de dose produit à la surface extérieure est compris entre 0,5 et 80 Gray par minute de préférence de 10 à 30 Gray/min.

L'invention concerne également un procédé d'irradiation utilisant le système décrit ci-dessus caractérisé en ce qu'une source de rayons X est insérée dans une tête d'applicateur puis que la source de rayons X est activée pour générer une énergie de 30 à 80 kV, plus précisément de 50 à 70 kV, et un débit de dose compris entre 0,5 et 80 Gray par minutes de préférence de 10 à 30 Gray/min à la surface extérieure de la tête d'application.

L'invention peut être utilisée dans un procédé de traitement par radiothérapie de contact, préférentiellement per-opératoire, utilisant le système décrit ci-dessus caractérisé en ce qu'il comprend le choix d'un applicateur de diamètre adapté à la cavité à traiter, le positionnement de la tête d'applicateur en contact des tissus de la cavité à traiter l'activation d'une source de rayons X pour générer une énergie de 30 à 80 kV pendant une durée comprise entre 20 secondes et 40 minutes, de préférence de 1 à 4 minutes de sorte à administrer une dose comprise entre 18 et 22 gray à la surface extérieure de la tête d'application.

Avantageusement, la dose administrée à 1 cm de profondeur du tissu est supérieure à 30%, avantageusement 40% de la dose à la surface extérieure de la tête d'application.

Les dessins ci-joints sont donnés à titre d'exemples et ne sont pas limitatifs de l'invention. Ils représentent seulement un mode de réalisation de l'invention et permettront de la comprendre aisément.
La figure 1 est une vue en perspective de l'applicateur selon l'invention.

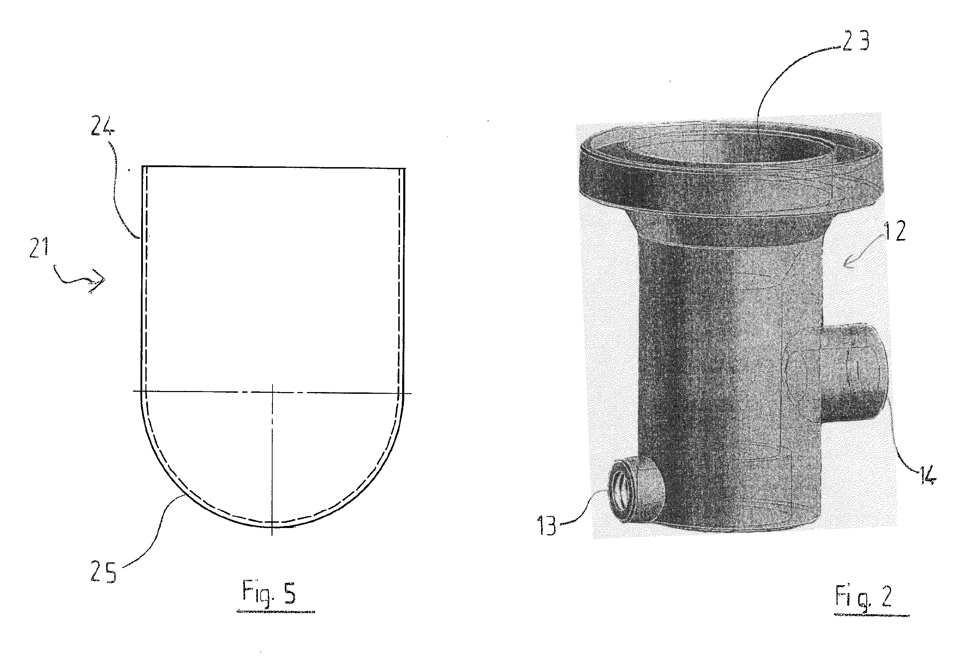

La figure 2 illustre un adaptateur d'un applicateur selon l'invention permettant de connecter l'applicateur à bras articulé mobile.

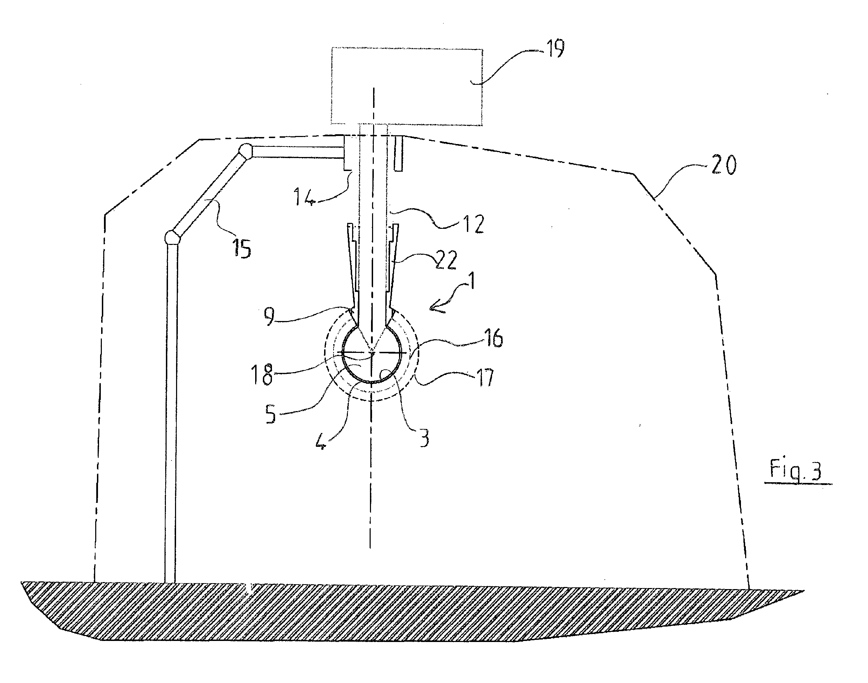

La figure 3 représente une vue d'ensemble d'un système de radiothérapie de contact per opératoire selon l'invention.

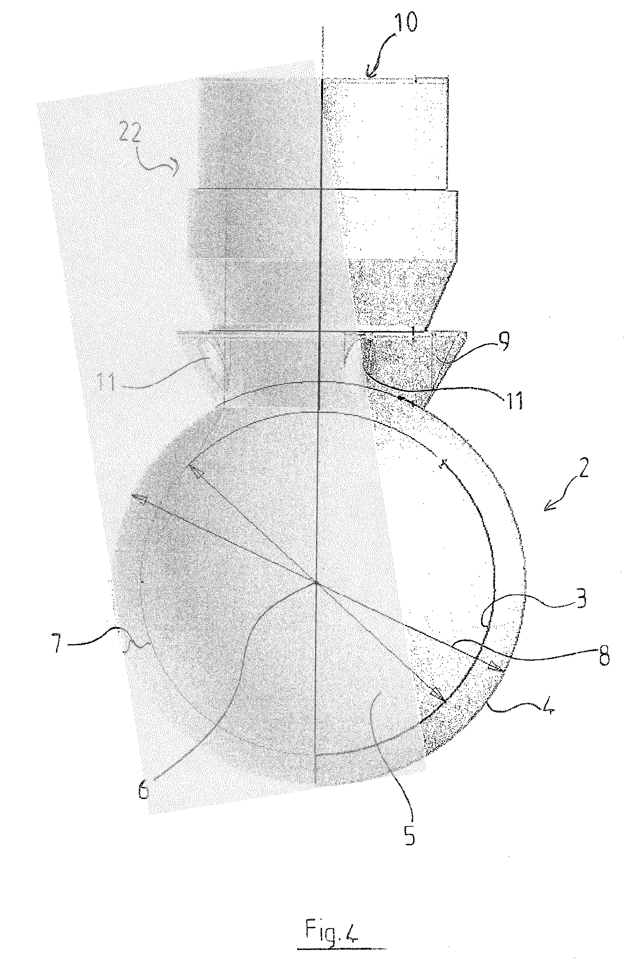

La figure 4 est une vue de côté de l'applicateur selon la figure 1.
La figure 5 illustre un filtre pour la source de rayons X.

L'invention concerne un jeu d'applicateurs pour la radiothérapie de contact. Le jeu d'applicateurs 1 comprend plusieurs applicateurs 1. Les applicateurs 1 du jeu sont de forme similaire. La description ci-après faite pour un applicateur 1 est avantageusement applicable à l'ensemble des applicateurs 1 du jeu.

L'applicateur 1 comprend une tête 2 d'application. Cette tête 2 d'application est destinée à être en contact d'une cavité d'un tissu vivant. En effet, la radiothérapie de contact per-opératoire permet d'irradier le tissu périphérique d'une tumeur qui a été retirée au cours d'une opération chirurgicale. La tête 2 est choisie par l'opérateur pour qu'elle soit en contact avec le lit d'exérèse. Le diamètre 8 de la tête 2 correspondant sensiblement au diamètre de la tumeur retirée et aux tissus environnants retirés. La tête 2 d'application est formée par une surface extérieure 4 destinée à être en contact avec la cavité à irradier et par une surface intérieure 3 définissant un volume intérieur 5. Les différents applicateurs 1 du jeu d'applicateurs selon l'invention sont de diamètres différents.

La tête 2 d'application est préférentiellement sphérique. On entend par sphérique que la surface extérieure 4 de la tête 2 correspond sensiblement à au moins une portion de sphère. La tête 2 est non déformable, elle est rigide.

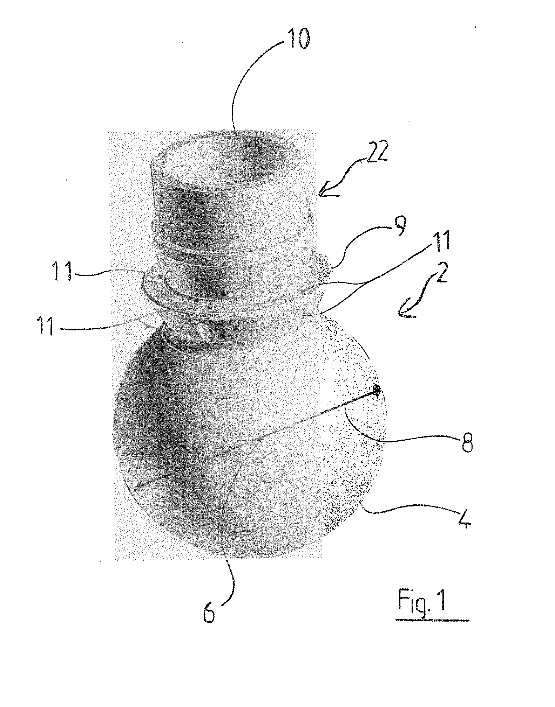

Tel que représenté à la figure 1, la tête 2 d'application est une sphère munie, au niveau d'une calotte supérieure, d'une ouverture 10. La tête 2 d'application est une portion de sphère, préférentiellement d'un secteur angulaire d'au moins 300°.

La tête 2 d'application est avantageusement creuse de sorte à recevoir une source de rayons X 18, préférentiellement en passant au travers de l'ouverture 10. La tête 2 d'application comprend avantageusement dans son volume intérieur 5 un gaz plus préférentiellement de l'air. Elle n'est préférentiellement pas remplie de liquide ou de fluide tel que de l'eau ou autre matériau de remplissage. Préférentiellement, la source de rayons X 18 est disposée au centre 6 de la tête 2. La précision du positionnement de la source de rayons X 18 est de l'ordre du millimètre. Le positionnement de la source de rayons X 18 au centre 6 de la tête 2 contribue avantageusement à produire une dose de rayons X homogène et constante à la surface extérieure 4. A titre d'exemple, en fonction du diamètre 8 de l'applicateur 1, un adaptateur 12, permettant de relier l'applicateur 1 avantageusement à un bras articulé de fixation 15, est de longueur différente. Selon une autre possibilité alternative ou additionnelle, ce peut être un support préférentiellement dénommé manche 22 décrit ci-après qui est de taille variable en fonction du diamètre 8 de la tête 2 de l'applicateur 1. Ces moyens permettent de s'assurer de l'emplacement central de la source de rayons X 18 dans la tête 2 de chaque applicateur et ceci malgré le fait que la tête 2 est creuse.

On entend par diamètre 8 la distance séparant deux points de la surface extérieure 4 de la tête 2 d'application passant par le centre 6 de la tête 2.

A titre d'exemple, le diamètre 8 des applicateurs 1 varie entre 2 et 6 centimètres.

Au moins une zone de la tête 2 est apte à être traversée par les rayons X. Cette zone est formée d'un matériau dont le coefficient d'absorption permet le passage du faisceau de rayons X. La zone de la tête 2 recevant les rayons X et apte à être traversée par ces derniers représente sensiblement un secteur angulaire d'au moins 300°, préférentiellement 310° de la tête 2, soit sensiblement toute la surface sphérique de la tête 2.

Selon l'invention, l'épaisseur 7 de la tête 2 d'application et plus précisément de la zone de la tête 2 d'application destinée à être traversée par des rayons X varie en fonction du diamètre 8 de la tête 2.

On entend par épaisseur 7, la distance séparant la surface intérieure 3 et la surface extérieure 4 de l'applicateur 1.

Préférentiellement, l'épaisseur 7 varie entre 1 et 3,5 millimètres, plus précisément entre 1 et 2,5 millimètres alors que le diamètre de la tête 2 varie de 2 à 7 cm plus précisément entre 2 et 6 cm; le volume intérieur 5 a un diamètre compris entre 1,8 et 6,5 cm.

Un des avantages d'une tête 2 creuse, dont le volume intérieur 5 est majoritairement rempli de gaz, notamment d'air, est d'améliorer nettement le débit de dose à la surface extérieure 4. Le débit de dose correspond à la dose de radiation reçu pour une unité de temps donnée. Plus le débit de dose est élevé, plus la dose à administrée est délivrée rapidement et la durée d'irradiation est diminuée. A titre d'exemple préféré, le débit de dose est de10 à 30 Gray pour une source de rayons X 18 de 50 kV.

Le matériau destiné à former la tête 2 est avantageusement un plastique présentant des propriétés de biocompatibilité. Le plastique choisi est préférentiellement à haute densité. A titre d'exemple, un plastique type polyamide tel du Polyhexaméthylène adipamide ou nylon® ou un plastique apte à être stérilisé préférentiellement au gaz. De manière préférée, l'applicateur 1, plus préférentiellement la tête 2, est à usage unique. Selon une possibilité, la tête 2 de l'applicateur 1 est en matériau transparent de sorte à visualiser son positionnement dans une cavité.

Le matériau constituant la tête 2 d'application et plus spécifiquement la zone de la tête 2 traversée par les rayons X possède avantageusement un coefficient d'absorption inférieur à 5% du débit de dose.

L'épaisseur 7 est préférentiellement différente pour les différents applicateurs 1.

Selon l'invention, plus l'applicateur 1 a un diamètre 8 important plus l'épaisseur 7 est faible et inversement.

La variation de l'épaisseur 7 en fonction du diamètre 8 est destinée à contrôler la dose de rayons X produite à la surface extérieure 4 de l'applicateur 1 et d'optimiser également le rendement en profondeur. Classiquement, plus le diamètre 8 de la tête 2 est faible, moins bon est le rendement en profondeur. Grâce à l'applicateur 1 selon l'invention, plus le diamètre 8 est faible, plus l'épaisseur 7 est grande, de sorte à maintenir un rendement en profondeur satisfaisant.

Le dispositif selon l'invention est particulièrement adapté pour la radiothérapie de contact. L'objectif est d'obtenir un rendement dose profondeur satisfaisant sur sensiblement 1 cm de profondeur de tissu à traiter. La profondeur s'entend entre la surface extérieure 4 de la tête en s'enfonçant dans le tissu à traiter. Selon l'invention, il est recherché d'obtenir un rapport entre la dose à la surface extérieure 4 de la tête et la dose à 1 cm de profondeur supérieur à 0,3, plus préférentiellement compris entre 0.4 et 0.5. Au-delà de cette profondeur, la dose de radiation est minime de sorte à protéger les structures environnantes telles le coeur, les côtes, le poumon...

Selon l'invention, quelle que soit la cavité à irradier, c'est-à-dire quel que soit le diamètre de l'applicateur 1, la dose de rayons X produite à la surface extérieure 4 de la zone de la tête 2 est homogène.

La dose de rayons X produite à la surface extérieure 4 de la tête 2 d'application est comprise entre 18 et 22 Gray, préférentiellement de l'ordre de 20 Gray. On entend par dose de rayons X, la dose qui est absorbée par le tissu de la cavité au contact de la surface extérieure 4 de la tête 2.

Le rendement en profondeur est contrôlé et sensiblement identique quel que soit le diamètre de l'applicateur 1. Préférentiellement, pour une cavité de tissu, on mesure une dose absorbée de 6 à 9 Gray, plus précisément de 7 à 9 Gray, à une distance de 10 millimètres. Par exemple, à une distance de 5 millimètres de la surface extérieure 4, la dose absorbée est de 10 à 13 Gray.

Selon un mode de réalisation, l'épaisseur 7 peut être constante sur toute la zone de la tête 2 traversée par les rayons X ou elle peut être différente. Si l'épaisseur 7 varie sur la zone traversée par les rayons X, cette variation peut être effectuée par l'adjonction d'une pièce additionnelle dans le volume intérieur 5 ou bien simplement par une variation de l'épaisseur 7. La variation d'épaisseur 7 le long du pourtour de la zone de la tête 2 traversée par les rayons X est particulièrement avantageuse lorsque la source de rayons X utilisée n'est pas parfaitement isotropique. Dans ce cas là, la dose n'est pas distribuée uniformément à la surface extérieure 4 de la tête 2 d'application. Le rendement dose - profondeur n'est pas non plus constant. Il est donc préféré d'augmenter ou de réduire l'épaisseur 7. Pour une tête 2 en plastique ayant une épaisseur 7 de base de 2,5mm, la dose sera diminuée ou augmentée de 10% par millimètre d'épaisseur respectivement ajouté ou retiré.

Selon une possibilité, un cache additionnel peut être placé dans le volume intérieur 5. Ce cache additionnel peut être utilisé par exemple pour bloquer le faisceau de rayons X sur un secteur angulaire correspondant par exemple à la direction d'un organe à préserver. Le cache additionnel peut être en différent matériau dont du métal ou du plastique.

Avantageusement, le volume intérieur 5 permet également de recevoir des moyens divers tels que une diode thermo luminescente ou un transistor à effet de champ de sorte à mesurer la radiation délivrée indépendamment du générateur de rayons X. Avec une simple mesure sans stérilisation additionnelle la dose délivrée est connue en fonction de l'épaisseur 7 de la tête 2 et du coefficient d'absorption du matériau utilisé.

Selon l'invention, la source de rayons X 18 est isotrope, préférentiellement la source de rayons X 18 émet dans les trois dimensions suivant un secteur angulaire d'au moins 300°, préférentiellement 310°. Préférentiellement, la source de rayons X 18 émet un faisceau avec une puissance de 50 à 70 kilovolts. Le faisceau est préférentiellement polychrome.

Selon un mode de réalisation de l'invention, la tête 2 de l'applicateur 1 est reliée à un adaptateur 12, préférentiellement par un manche 22. Ce manche 22 de forme préférentiellement cylindrique creux permet le passage de la source de rayons X 18. Le manche 22 est connecté à la tête 2 au niveau de l'ouverture 10 par exemple de manière fixe. Préférentiellement, au niveau de la jonction entre le manche 22 et la tête 2, l'applicateur 1 comprend un rebord 9. Le rebord 9 est avantageusement circulaire, préférentiellement entourant l'ouverture 10. Le rebord 9 forme une margelle. Le rebord 9 forme une butée au niveau de laquelle vient en contact le pourtour des tissus de la cavité à irradier. Cette disposition présente l'avantage de fixer l'applicateur dans la cavité ainsi que de protéger des rayons X les tissus à risques qui sont positionnés pardessus ce rebord 9. Avantageusement, Le rebord 9 matérialise la limite angulaire du faisceau de rayons X. De cette manière, la cavité d'exérèse est irradiée par une dose homogène même à proximité du rebord 9. Selon un mode de réalisation, le rebord 9 est mobile relativement au centre de la tête 2 d'application. Le rebord 9 est avantageusement mobile au moins en translation le long du manche 22. Il peut également être mobile en rotation autour du manche 22. La mobilité en translation du rebord 9 par rapport au manche 22 permet une modularité de l'applicateur permettant d'enfoncer plus profondément dans le tissu à traiter la tête 2 d'application ; le rebord 9 restant à l'extérieur de la cavité d'exérèse. Ceci est particulièrement avantageux lorsque des moyens de fixation tels que décrits ci-après sont formés au niveau dudit rebord 9.

Avantageusement, l'applicateur 1 comprend des moyens de fixation destinés à permettre le maintien de l'applicateur 1 dans la cavité à irradier. Les moyens de fixation coopèrent avec le pourtour de la cavité à irradier. A titre d'exemple, les moyens de fixation comprennent des trous 11 au travers desquels des fils de suture peuvent être passés. Préférentiellement, les trous 11 sont formés dans le rebord 9.

L'applicateur 1 est fixé à un adaptateur 12 par des moyens d'accrochage notamment des moyens de serrage 13. L'adaptateur 12 comprend des moyens de connexion 14 à un bras articulé 15.

L'adaptateur 12 est creux pour permettre le passage de la source de rayons X 18 suivant une direction 23.

Avantageusement, le bras articulé 15, l'adaptateur 12 et l'applicateur 1 sont couverts par un champ stérile 20. A titre préféré, le champ stérile 20 est perforé, par exemple découpé au ciseau stérile, pour laisser passer le tube du générateur 19 dans l'applicateur 1 sans compromettre la bonne stérilité de l'ensemble de la procédure. Le générateur de rayons X 19 est à l'extérieur du champ stérile 20.

Selon une variante préférée de l'invention, la source de rayons X 18 comporte un filtre 21 destiné à filtrer le rayonnement. Ce filtre 21 est préférentiellement amovible. A titre préféré, le filtre 21 est en métal tel l'aluminium d'une épaisseur de 0,2 à 2,5 millimètres, plus précisément de 0,2 à 0,9 millimètres, préférentiellement 0,3 à 0,8 millimètres. Il est préféré d'utiliser un filtre 21 d'épaisseur inférieure à 0,4 mm car au-delà l'effet de réduction des rayons de faible énergie, de grande longueur d'onde, c'est-à-dire l'effet de durcissement du faisceau, est faible et le débit de dose est fortement diminué ce qui nécessite d'augmenter la durée d'exposition.

Ce filtre 21 peut être utilisé dans un système pour la radiothérapie de contact de manière indépendante du dispositif de radiothérapie selon l'invention.

Ce filtre 21 permet d'améliorer le rendement en profondeur du faisceau de rayons X en absorbant les radiations de basse énergie. Le faisceau de rayons X est durci.

A titre d'exemple, le filtre 21 permet d'obtenir un rendement en profondeur de 50% à 7 millimètres de profondeur de tissu vivant par rapport à 50% à 5 millimètres en l'absence du filtre 21. En parallèle, la dose est diminuée de 30%, il est donc nécessaire d'augmenter la durée d'irradiation de 30%.

Le filtre 21 présente une forme complémentaire de la source de rayons X 18. Le filtre 21 a une forme de manchon composé d'une portion cylindrique 24 avec une extrémité débouchant et dont l'autre extrémité est obturée par une pièce arrondie 25, préférentiellement, une calotte en demi-sphère. Avantageusement, l'épaisseur du filtre 21 est fonction de l'angle d'incidence des rayons X à sa surface.

Le filtre 21 est tout particulièrement utile pour les applicateurs 1 de diamètre faible, c'est-à-dire inférieur à 4,5 centimètres.

A titre d'exemple, avec l'invention, on peut irradier une cavité avec une dose à la surface extérieure 4 de l'applicateur 1 de l'ordre de 20 Gray en une fois pendant une durée de l'ordre de 1 à 4 minutes, préférentiellement 2 minutes, avec un rendement en profondeur satisfaisant à 10 millimètres de la surface extérieure 4 de l'applicateur 1.

### REFERENCES

- 1.: Applicateur
- 2.: Tête
- 3.: Surface intérieure
- 4.: Surface extérieure
- 5.: Volume intérieur
- 6.: Centre de la tête
- 7.: Epaisseur
- 8.: Diamètre
- 9.: Rebord
- 10.: Ouverture
- 11.: Trous
- 12.: Adaptateur
- 13.: Moyen de serrage
- 14.: Moyen de connexion au bras
- 15.: Bras articulé
- 16.: Zone à une distance de 5 millimètres de la surface extérieure
- 17.: Zone à une distance de 10 millimètres de la surface extérieure
- 18.: Source de rayons X
- 19.: Générateur
- 20.: Champ stérile
- 21.: Filtre
- 22.: Manche
- 23.: Direction d'introduction de la source de rayons X
- 24.: Portion cylindrique
- 25.: Pièce arrondie

## Revendications

1. Système pour la radiothérapie de contact comprenant un dispositif et une source de rayons X (18) émettant un faisceau isotrope,
ledit dispositif comprenant un jeu d'applicateurs (1) dont chaque applicateur (1) comprend une tête (2) sphérique et creuse, de diamètre (8) différent, dont la surface extérieure (4) est apte à être en contact avec une cavité d'un tissu vivant et dont la surface intérieure (3) définit un volume intérieur (5) recevant la source de rayons X (18),
dans lequel au moins une zone de la tête (2) est destinée à être traversée par des rayons X et l'épaisseur (7) de ladite zone de chaque applicateur (1) est comprise entre 1 et 3,5 millimètres et est une fonction du diamètre (8) de la tête (2), de sorte que plus le diamètre est faible, plus l'épaisseur est grande de sorte à maintenir un rendement en profondeur quel que soit le diamètre de la tête, ladite zone de la tête étant configurée de sorte que a) la dose de rayons X produite à la surface extérieure (4) est comprise entre 18 et 22 Gray administrée pendant une durée de 1 à 4 minutes, ou b) le débit de dose produite à la surface extérieure (4) est de 0,5 à 80 Gray par minute.

2. Système selon la revendication précédente, dans lequel l'épaisseur (7) de ladite zone de la tête (2) de chaque applicateur (1) est comprise entre 1 et 2,5 millimètres.

3. Système selon l'une quelconque des revendications précédentes, dans lequel le diamètre (8) de la tête (2) de chaque applicateur (1) est compris entre 2 et 7 centimètres.

4. Système selon l'une quelconque des revendications précédentes, dans lequel le diamètre (8) de la tête (2) de chaque applicateur (1) correspond à un secteur angulaire d'au moins 300°.

5. Système selon l'une quelconque des revendications précédentes, dans lequel la tête (2) de chaque applicateur (1) comprend une matière plastique apte à être stérilisée.

6. Système selon l'une quelconque des revendications précédentes, dans lequel chaque applicateur (1) comprend un rebord (9) en partie supérieure de la tête (2) d'application.

7. Système selon la revendication précédente, dans lequel chaque applicateur (1) comprend un manche (22) s'étendant depuis la tête (2) d'application, ledit manche (22) recevant le rebord (9) configuré pour être mobile en translation le long du manche (22).

8. Système selon l'une quelconque des revendications précédentes, dans lequel chaque applicateur (1) comprend des moyens de fixation de la tête (2) dans la cavité d'un tissu vivant.

9. Système selon la revendication précédente en combinaison avec l'une des revendications 6 ou 7, dans lequel les moyens de fixation sont formés par le rebord (9) muni de trous (11), et formant une calotte sur la tête (2) de l'applicateur (1).

10. Système selon l'une quelconque des revendications précédentes, dans lequel la tête (2) de chaque applicateur (1) comprend une ouverture (10) apte à permettre l'introduction de la source de rayons X (18) dans le volume intérieur (5).

11. Système selon l'une quelconque des revendications précédentes, dans lequel la source de rayons X (18) est polychrome et émet une puissance de 30 à 80 kV, préférentiellement de 50 à 70 kV.

12. Système selon l'une quelconque des revendications précédentes, dans lequel la source de rayons X (18) est disposée au centre (6) de la tête (2) de l'applicateur (1).

13. Système selon l'une quelconque des revendications précédentes, dans lequel le volume intérieur (5) de la tête (2) d'application est rempli de gaz.

14. Système selon l'une quelconque des quatre revendications précédentes, comprenant un filtre (21) en aluminium agencé au niveau de la source de rayons X (18).

15. Système selon la revendication précédente, dans lequel le filtre (21) a une épaisseur comprise entre 0,2 à 2,5 millimètres.

## Patentansprüche

1. System für die Kontaktstrahlentherapie, das eine Vorrichtung und eine Röntgenstrahlenquelle (18), die ein isotropes Strahlungsfeld emittiert, umfasst,
wobei die besagte Vorrichtung einen Satz Applikatoren (1) umfasst, von denen jeder Applikator (1) einen kugelförmigen und hohlen Kopf (2) mit unterschiedlichem Durchmesser (8) umfasst, dessen Außenfläche (4) dazu geeignet ist, mit einem Hohlraum eines lebenden Gewebes in Kontakt zu sein, und dessen Innenfläche (3) ein Innenvolumen (5) definiert, das die Röntgenstrahlenquelle (18) aufnimmt,
wobei mindestens eine Zone des Kopfs (2) dazu vorgesehen ist, von Röntgenstrahlen durchströmt zu werden, und die Dicke (7) der besagten Zone jedes Applikators (1) zwischen 1 und 3,5 Millimeter liegt und von dem Durchmesser (8) des Kopfs (2) abhängt, so dass je kleiner der Durchmesser ist, desto größer die Dicke ist, um eine relative Tiefendosis ungeachtet des Durchmessers des Kopfs aufrechtzuerhalten, wobei die besagte Zone des Kopfs derart konfiguriert ist, dass a) die Röntgenstrahlendosis, die auf der Außenfläche (4) hervorgerufen wird, zwischen 18 und 22 Gray liegt, die über einen Zeitraum von 1 bis 4 Minuten verabreicht wird, oder b) die Dosisleistung, die auf der Außenfläche (4) hervorgerufen wird, 0,5 bis 80 Gray pro Minute ist.

2. System gemäß dem vorhergehenden Anspruch, wobei die Dicke (7) der besagten Zone des Kopfs (2) jedes Applikators (1) zwischen 1 und 2,5 Millimeter liegt.

3. System gemäß einem der vorhergehenden Ansprüche, wobei der Durchmesser (8) des Kopfs (2) jedes Applikators (1) zwischen 2 und 7 Zentimeter liegt.

4. System gemäß einem der vorhergehenden Ansprüche, wobei der Durchmesser (8) des Kopfs (2) jedes Applikators (1) einem Winkelsektor von mindestens 300° entspricht.

5. System gemäß einem der vorhergehenden Ansprüche, wobei der Kopf (2) jedes Applikators (1) einen Kunststoff umfasst, der dazu geeignet ist, sterilisiert zu werden.

6. System gemäß einem der vorhergehenden Ansprüche, wobei jeder Applikator (1) einen Rand (9) im oberen Teil des Applikationskopfs (2) umfasst.

7. System gemäß dem vorhergehenden Anspruch, wobei jeder Applikator (1) eine Hülse (22) umfasst, die sich von dem Applikationskopf (2) erstreckt, wobei die besagte Hülse (22) den Rand (9) aufnimmt, der dazu konfiguriert ist, entlang der Hülse (22) translatorisch beweglich zu sein.

8. System gemäß einem der vorhergehenden Ansprüche, wobei jeder Applikator (1) Mittel zur Fixierung des Kopfs (2) in dem Hohlraum eines lebenden Gewebes umfasst.

9. System gemäß dem vorhergehenden Anspruch in Kombination mit einem der Ansprüche 6 oder 7, wobei die Fixierungsmittel von dem Rand (9) gebildet werden, der mit Löchern (11) versehen ist und auf dem Kopf (2) des Applikators (1) eine Kappe bildet.

10. System gemäß einem der vorhergehenden Ansprüche, wobei der Kopf (2) jedes Applikators (1) eine Öffnung (10) umfasst, die dazu geeignet ist, die Einführung der Röntgenstrahlenquelle (18) in das Innenvolumen (5) zu ermöglichen.

11. System gemäß einem der vorhergehenden Ansprüche, wobei die Röntgenstrahlenquelle (18) polychrom ist und eine Leistung von 30 bis 80 kV, vorzugsweise 50 bis 70 kV emittiert.

12. System gemäß einem der vorhergehenden Ansprüche, wobei die Röntgenstrahlenquelle (18) in der Mitte (6) des Kopfs (2) des Applikators (1) angeordnet wird.

13. System gemäß einem der vorhergehenden Ansprüche, wobei das Innenvolumen (5) des Applikationskopfs (2) mit Gas gefüllt ist.

14. System gemäß einem der vorhergehenden Ansprüche, das einen Filter (21) aus Aluminium umfasst, der auf Höhe der Röntgenstrahlenquelle (18) eingerichtet ist.

15. System gemäß dem vorhergehenden Anspruch, wobei der Filter (21) eine Dicke hat, die zwischen 0,2 und 2,5 Millimeter liegt.

## Claims

1. System for contact radiotherapy comprising a device and an X-ray source (18)emitting an isotropic beam, said device comprising a set of applicators (1), of which each applicator (1) comprises a spherical and hollow head (2), of a different diameter (8), the outer surface (4) of which is able to be in contact with a cavity of living tissue and the inner surface (3) of which defines an interior space (5) receiving the X-ray source (18),
wherein at least one area of the head (2) is intended to be crossed by X-rays and the thickness (7) of said area of each applicator (1) is comprised between 1 and 3.5 millimetres and depends on the diameter (8) of the head (2), so that the smaller the diameter, the larger the thickness so as to maintain a yield in depth regardless of the diameter of the head, said area of the head being configured so that a) the X-ray dose produced at the outer surface (4) is comprised between 18 and 22 Grays administered for a period from 1 to 4 minutes, or b) the dose rate produced at the outer surface (4) is from 0.5 to 80 Grays per minute.

2. The system according to the preceding claim, wherein the thickness (7) of said area of the head (2) of each applicator (1) is comprised between 1 and 2.5 millimetres.

3. The system according to any of the preceding claims, wherein the diameter (8) of the head (2) of each applicator (1) is comprised between 2 and 7 centimetres.

4. The system according to any of the preceding claims, wherein the diameter (8) of the head (2) of each applicator (1) corresponds to an angular sector of at least 300°.

5. The system according to any of the preceding claims, wherein the head (2) of each applicator (1) comprises a plastic material able to be sterilised.

6. The system according to any of the preceding claims, wherein each applicator (1) comprises a rim (9) in the upper portion of the application head (2).

7. The system according to the preceding claim, wherein each applicator (1) comprises a handle (22) extending from the application head (2), said handle (22) receiving of the rim (9) configured so as to be translationally mobile along the handle (22).

8. The system according to any of the preceding claims, wherein each applicator (1) comprises means for attaching the head (2) in the cavity of a living tissue.

9. The system according to the preceding claim combined with one of claims 6 or 7, wherein the attachment means are formed by the rim (9) provided with holes (11), and forming a cap on the head (2) of the applicator (1).

10. The system according to any of the preceding claims, wherein the head (2) of each applicator (1) comprises an aperture (10) able to allow introduction of the X-ray source (18) into the interior space (5).

11. The system according to any of the preceding claims, wherein the X-ray source (18) is polychromatic and emits a power of 30 to 80 kV, preferentially from 50 to 70 kV.

12. The system according to any of the preceding claims, wherein the X-ray source (18) is positioned in the centre (6) of the head (2) of the applicator (1).

13. The system according to any of the preceding claims, wherein the interior volume (5) of the application head (2) is filled with gas.

14. The system according to any of the four preceding claims, comprising an aluminium filter (21) laid out at the X-ray source (18).

15. The system according to the preceding claim, wherein the filter (21) has a thickness comprised between 0.2 to 2.5 millimetres.
